# EUROPEAN PATENT SPECIFICATION

(11) **EP 3 493 690 B1**
(45) Date of publication and mention of the grant of the patent: **09.12.2020**
(21) Application number: 17758634.4
(22) Date of filing: 04.08.2017
(51) Int. Cl.: A41B 11/00, A41D 13/00, A61F 13/08, A41B 11/08, A41D 31/18

(54) **TUBULAR WEARABLE TEXTILE ARTICLE**
RÖHRENFÖRMIGER TRAGBARER TEXTILARTIKEL
ARTICLE VESTIMENTAIRE TEXTILE TUBULAIRE

(30) Priority: 05.08.2016 IT 201600082944
(43) Date of publication of application: 12.06.2019
(73) Proprietor: Revenge S.r.l., 25124 Brescia (IT)
(72) Inventor: LONGHI, Alberto, 25124 Brescia (IT)
(74) Representative: Pulieri, Gianluca Antonio
(86) International application number: PCT/IB2017/054795
(87) International publication number: WO 2018/025238

(56) References cited:
- WO-A1-2012/114209
- US-A1- 2013 160 189
- US-A1- 2014 082 815

## Description

The present invention relates to a wearable textile article, in particular a tubular wearable textile article to be worn during sports or, for example, in post-trauma rehabilitation activities.

It is known that during exercise, one might run into minor muscle injuries resulting in the need to support some body segments as they are weakened and/or sore.

To this end, in the field of wearable aids for sports or for rehabilitation, footwear and braces of elastic polyester (knee pads, calf compresses, wrist supports, etc.) are known, adapted to support or heat the muscle in performing physical activities.

However, these types of wearable aids are primarily intended to support the neuro-muscular apparatus, for example through local compression or in such a way as to restrict movements or otherwise so as to avoid worsening a neuro-muscular situation already compromised. Examples of these types of wearable aids are disclosed in US2013/160189A1, WO2012/114209A1, US2014/082815A1.

One of the objects of the present invention is to propose a wearable textile article able to improve support to the muscles, but also having an improvement function of the recovery time from trauma and also a preventive function.

Said object is achieved with a wearable textile article according to claim 1. The dependent claims describe preferred or advantageous embodiments of the textile article.

The features and the advantages of the textile article according to the invention shall be made readily apparent from the following description of preferred embodiments thereof, provided purely by way of a non limiting example, with reference to the accompanying figures, in which:
figure 1a shows an inner rear side of the textile article (i.e. the reverse of the textile article) intended to come into contact with the skin when worn by an individual, according to one embodiment of the invention;
figure 1b shows an outer rear side of the wearable textile article corresponding and opposite to the inner rear side of figure 1a;
figure 1c shows an embodiment of the textile article when worn on a leg, having a calf compress function, wherein the outer side of the wearable article has been intentionally removed in order to make the arrangement of the elastic bands around the calf visible;
figure 2a shows a plan view of the bands made of polymeric material according to a variant of the invention;
figure 2b shows a plan view of the bands made of polymeric material according to another variant of the invention;
figure 3 shows an embodiment of the textile article when worn on an upper limb, such as a sleeve having an elbow compress function, where the outer side of the wearable article has been intentionally removed in order to make the arrangement of the elastic bands around the upper limb visible;
figure 4 shows a plan view of the bands made of polymeric material according to another variant of the invention, for example used for a sleeve for an upper limb.

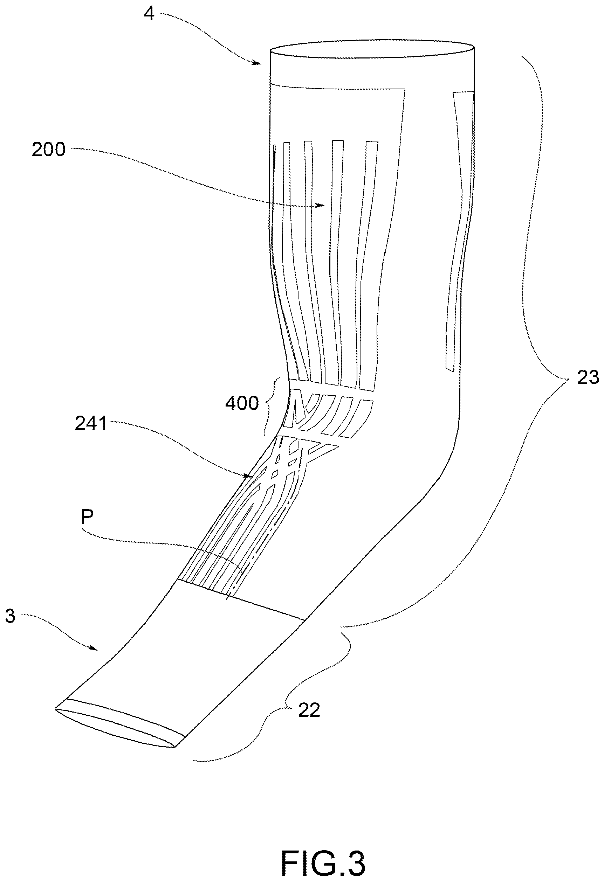

With reference to the above figures, reference numeral 1 indicates a wearable textile article for the assistance of the muscle function, in particular in performing physical activities. Such a wearable textile article is preferably a sleeve worn on the limbs, such as a calf compress or a sleeve worn on one of the upper limbs (figure 3), around the elbow, such as an elbow compress. The textile article 2 comprises a tubular element 20 made of fabric, having an inner side 20 suitable for coming into contact with a part of the human body and an outer side 21 opposite the inner side.

In one embodiment, the tubular element comprises an annular lower elastic strip 3 at the lower base 25 of the tubular element and an annular upper elastic strip 4 at the upper base 26 of the tubular element. In the case of a calf compress, the annular lower strip 3 is suitable for squeezing around the ankle and the upper elastic strip 4 is suitable for squeezing below the knee, when the textile article is worn. In the case of a sleeve worn around the upper limb, the annular lower strip 3 is suitable for squeezing around the forearm, preferably at about half the forearm and the upper elastic strip 4 is suitable for squeezing around the arm, above the elbow, preferably at about half the arm.

Preferably, moreover, the tubular element 2 inferiorly comprises a lower tapered region having a smaller annular diameter D than an upper region 23. For example, the lower tapered region 22 extends in the vicinity of the lower elastic strip 3 by a height less than half the total height of the tubular element, for example by a height less than one third of the total height H of the tubular element.

On the inner side 20 of tubular element 20 is applied a plurality of bands 200 made of polymeric material, suitable to be in skin contact with a contact region of the part of the human body. Bands 200 are arranged along paths P suitable to overlap with the paths of the subcutaneous nerve and/or muscle fibres of the human body part in contact with such bands 200, when the textile article is worn. In other words, the paths mainly follow an extension direction of the muscle fibres at which the textile article is worn. For example, in the case of a calf compress, the paths develop mainly along a direction that proceeds from the ankle to the knee when the calf compress is worn. In the case of a sleeve for the upper limb, the paths develop mainly along a direction that proceeds from the forearm towards the arm, or in any case from the wrist to the shoulder, and are suitable to arrange themselves in the anterior and antero-internal region of the limb. In a particularly advantageous manner, paths P of the bands overlap to the paths of the medial and lateral gastrocnemius and/or soleus muscle fibres muscles in the case of the calf compress, or to the paths of the muscle fibres of the biceps and/or radial and ulnar flexor muscle in the case of a sleeve for the upper limb (for example, an elbow compress), preferably on the radial and ulnar flexor digitorum muscle.

Unlike the prior art aids, such an arrangement of bands 200 along said paths P allows creating an effect of decompression of the body part in contact with the bands and a simultaneous stimulating effect of the muscle and/or nervous fibres at such a contact region.

Preferably, the polymeric material of which bands 200 are made is a silicone-based material, suitable to ensure the adhesion of the bands to the skin during the movement of the body part in contact therewith. Even more preferably, the bands are made of a silicone elastomer having a shore hardness A of between 5 and 30, preferably about equal to 14. Moreover, preferably, the silicone elastomer is suitable to adhere with the fabric of the tubular element and is resistant to washing (also to dry cleaning) and leaves no residues on the skin.

Preferably, bands 200 extend at least along a curved path P having a preferred longitudinal direction, for example a direction parallel to the generatrices of the tubular element 2, or a direction parallel to a median axis X of the tubular element. In one embodiment of the invention, bands 200 extend substantially throughout height H of the tubular element, along the preferred longitudinal direction X.

In a further embodiment, bands 200 extend for a given band length L from the lower region 22 of the tubular element to the upper region 23 of the tubular element in the direction of height H of the tubular element and for a given transverse width T in the direction perpendicular to length L. In an intermediate region 24 between said lower region 22 and said upper region 23, bands 200 are tapered towards the median axis X incident on the transverse width T.

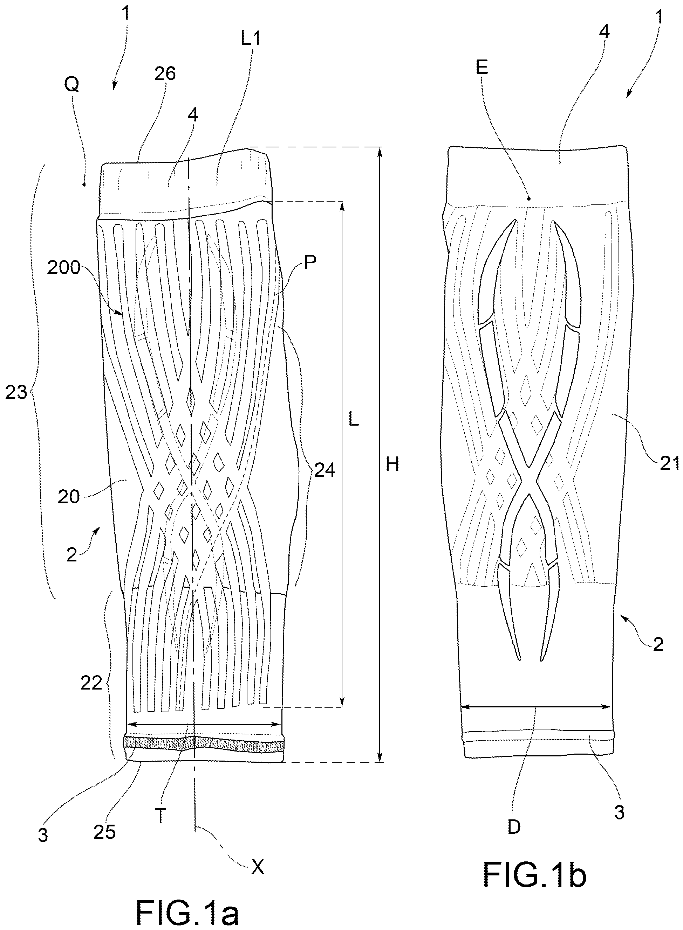

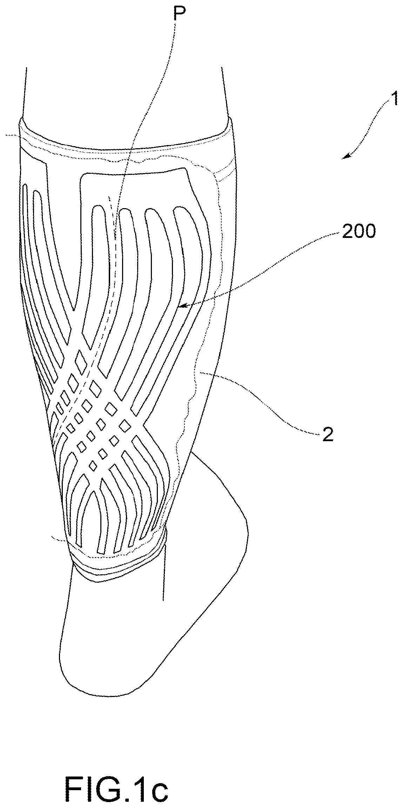

Preferably, the bands are arranged in a region of the tubular element intended to come into contact with a rear region of a limb of an individual, such as the calf in the case of a calf compress, or with a front region of the limb in the case of an upper limb (for example, in an elbow compress). Preferably, the bands are arranged only on a part of the entire lateral surface of the tubular element, more in particular only on half of the lateral surface of the tubular element. In other words, when the tubular element is arranged collapsed on a support plane Q (such as shown in figure 1a), it has an inner face L1 on which bands 200 are arranged and an opposite inner face L2 (not visible in figure 1a as it is resting against the support plane) free from bands 200. The opposite face L2 is intended to come into contact with the human body part not intended for muscular support (such as the front of the leg opposite the calf).

Preferably, on the outer surface of the tubular element (i.e. the surface not in contact with the body) there are no bands, so as not to compromise the general aesthetics of the article.

In this way, when the element is worn on the leg of an individual, the elastic bands 200 are in contact only with the back portion of the leg, that is, in the calf region, while in the front portion, on the side of the tibia, there are no elastic bands on the inner side or on the outer side of the tubular element 20. In the case of a sleeve for the upper limb, the elastic bands 200 are in contact only with the front portion of the limb, or in an embodiment variant, they are in contact with both the front area and the back area.

According to the invention, bands 200 are interconnected so as to form a weave or a lattice, preferably at the intermediate region 24.

In this variant, preferably, when the textile article is worn on the leg, the lattice of bands 200 is positioned in the lower region of the calf, to give more support to the muscle-tendon region. When the textile article is worn on the upper limb, the lattice of bands 200 is positioned in the region just below the elbow, to give more support to the muscle-tendon region of the joint.

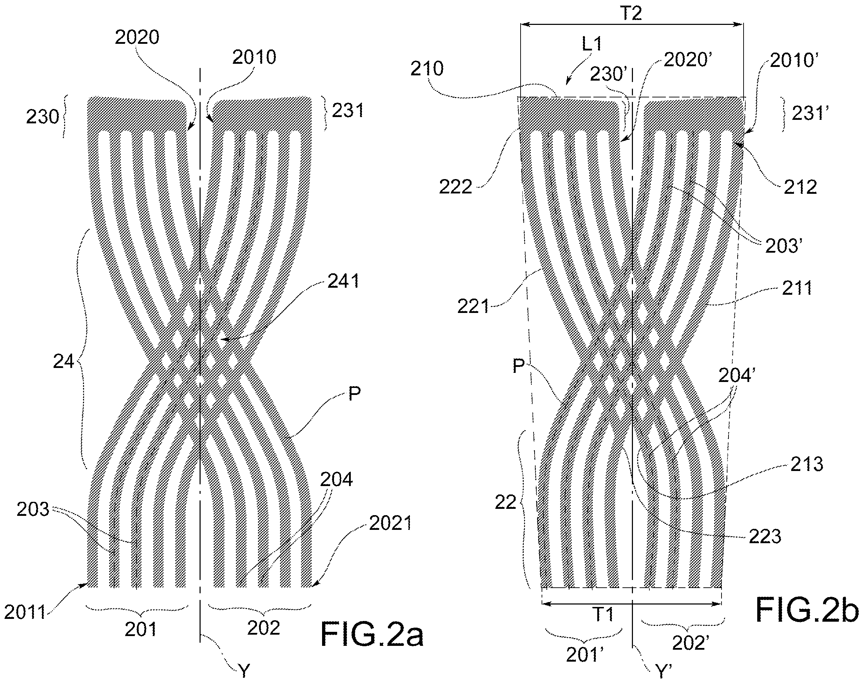

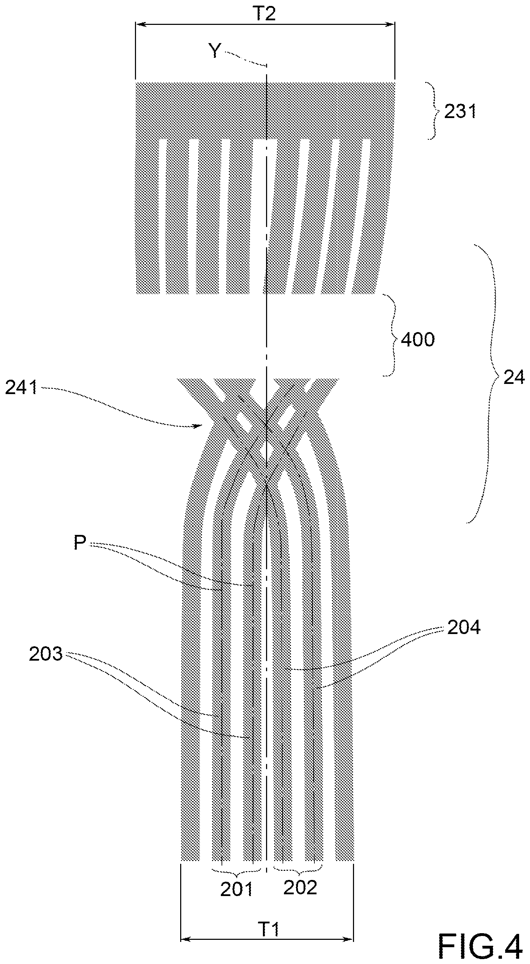

Two preferred embodiments of bands 200 are shown in figures 2a and 2b, which show the bands seen in a planar projection of the lateral surface of the tubular element. A further embodiment of bands 200 is shown in figure 4.

According to the invention, bands 200 comprise a first group of bands 201; 201' comprising a first plurality of bands spaced apart and arranged along first parallel paths 203; 203' and a second group of bands 202; 202' comprising a second plurality of bands spaced apart and arranged along second parallel paths 204; 204'. These second parallel paths 204; 204' are arranged substantially specular with respect to the first parallel paths 203; 203' with respect to a longitudinal axis of symmetry Y; Y' parallel to the generatrices of the tubular element. Such an axis of symmetry Y; Y' is preferably coincident with the median axis X. In other words, the axis of symmetry Y; Y' coincides with the median axis of the planar projection of the tubular element that shows face L1 on which bands 200 are arranged.

Preferably, the first 203; 203' and the second 204; 204' parallel paths have a curved shape. Advantageously, the first 203; 203' and second 204; 204' parallel paths intertwine to form an overall "X" shape (e.g. as shown in figures 2a, 2b and 4). As a result, a lattice of bands 241 is formed at the intermediate region 24, preferably having a rhomboid shape.

Preferably, the first 201; 201' and the second 202; 202' group of bands consist each of four spaced bands.

In one embodiment, for example shown in figure 2b and in figure 4, in the vicinity of the lower region 22, bands 200 extend in a direction perpendicular to the generatrices of the tubular element by a smaller transverse width T1. In the vicinity of the upper region 23, bands 200 extend in a direction perpendicular to the generatrices of the tubular element by a larger transverse width T2 than the lower transverse width T1. In other words, the outer envelope 210 of bands 200 tapers downwards toward the median axis X (i.e. the axis of symmetry Y'). By outer envelope it is meant the convex geometric figure obtained by joining the tangents to the outermost points of the plurality of bands 200. For example, in the case shown in figure 2b, the outer envelope has a square shape, more precisely it is an isosceles trapezoid in which the minor base is located in the vicinity of the lower base 25 of the tubular element and the major base is located in the vicinity of the upper base 26 of the tubular element 2.

In a preferred embodiment variant, the fabric of which the tubular element is made comprises carbon fibres, which improve the breathability of the textile article and at the same time ensures the maintenance of the body temperature.

Preferably, the thickness of bands 200, measured as the maximum distance between the inner side 20 of the tubular element and the top of each band on a transverse plane secant in a perpendicular manner the lateral surface of the tubular element, is of between 0.1 mm and 2 mm, preferably 0.4 mm; in this way, comfort is not affected when the article is worn and at the same time, the effectiveness of the function of the bands is ensured.

Preferably, the bands have a cross-section substantially shaped as a semicircle (or semicircle squashed on the apex), in which the straight base of the semicircle is joined to the inner side 20, while the apex of semicircle projects from the inner side. Therefore, the transverse thickness of the bands corresponds to the distance between the base of the semicircle and the apex.

Preferably, the bands belonging to the first group of bands 201; 201' have upper end portions 2010 joined together and lower end portions 2011 not joined together. Likewise, the bands belonging to the second group of bands 202; 202' have upper end portions of second group 2020 joined together and lower end portions of second group 2021 not joined together.

At the upper end portions 2010, 2020, each group of bands 201, 202; 201', 202' is joined to a reinforcement band 230, 231; 230', 231' that mutually joins the bands of each group of band 201, 202.

In the embodiment variant shown in figure 2b, an outer band 211, 221 of the bands of each group of band 201', 202' extends along a path that has a shorter length than the other bands of the same group. More in particular, such an outer band has a band end 212, 222 joined to the upper end portions 2020', 2010' of the other bands of the same group of band and a lower band end 213, 223 ending by joining an intermediate portion of one of the remaining bands of the same group of band. Advantageously, this configuration allows obtaining the inferiorly tapered shaped of the bands in a simpler manner and with a reduced use of material.

In the embodiment variant shown in figure 4, preferably more suitable for a sleeve for an upper limb, the continuity of bands 200 is interrupted in an interrupted region 400, free from bands, in which there are no bands 200 and corresponding to the elbow region on the front side when the textile article is worn. In this way, advantageously, the bending movement of the forearm on the arm is not prevented or bothered. Preferably, the interrupted region 400, without bands 200, extends in the direction of the longitudinal axis of symmetry Y by a stretch of between 5 and 20% of the total length of the wearable article, preferably by a stretch equal to 10% of the total length (e.g. by about 3 cm).

Moreover, preferably, the intermediate region 400 free from bands is arranged just above the lattice of bands 241, in the direction that proceeds from the wrist to the shoulder.

It is clear that the wearable textile article described can also be implemented in the form of a sock or footwear and similar articles. In fact, a man skilled in the art can apply to the lower base of the tubular element the fabric portions necessary to achieve a sock shape to be worn on the foot of an individual.

Moreover, it is clear that the wearable article is not exclusively intended for human subjects, but may also be applied to animals, such as pets.

In addition, it should be noted that the thicknesses used and the consistency of the bands are specifically designed to not generate any constraint or pain during use and at the same time to exert a decompression on the underlying part, which causes an effect of improving circulation.

An advantageous production process for the implementation of the wearable article is described hereinafter.

One starts with a tubular element 2 woven in the desired yarn (for example a yarn comprising polyamide fibres, such as microfiber, carbon and elastane).

The tubular element 2 is then fitted on a template with the inner side facing outwards (i.e. is fitted upside down). The template is suitable for stretching the fabric of the tubular element so that a printing region of the lateral surface of the tubular element is essentially planar.

The desired pattern of the bands of polymeric material is then printed by a silk-screen printing technique on the printing region.

Preferably, the printing by a silk-screen printing technique involves positioning a shaped mask according to a negative of the desired pattern for bands 200. Thereafter, the polymeric material in liquid form (such as a silicone material) is spread on the mask, preferably by means of a spatula. Finally, once the mask has been removed, the wearable article is positioned in an oven at a temperature suitable for hardening (or curing) the polymeric material.

Innovatively, the wearable textile article according to the present invention allows assisting the muscular function without limiting the movements thereof, activating the body's natural healing process and leveraging the elasticity of the band of polymeric material with respect to that of the skin. The muscles and joints of the area subject to contact with the bands of polymeric material are in tension. This increases the space in the subcutaneous region where the initial lymph vessels, the capillaries and the different afferent and efferent receptors are located. One of the main benefits is the analgesia and pressure reduction and improved circulation.

In addition, the textile article according to the present invention allows an improved drainage of the muscle group and a constant stimulus to micro circulation, ideal to reduce the recovery time for muscle oedema, bruises, inflammation.

Advantageously, moreover, the presence of carbon fibres in the fabric, combined with the internal printing of the bands of polymeric material allows combining the benefits of carbon (i.e. bacteriostasis, high breathability, and temperature-regulating action) with the beneficial effects of the decompression-effect bands in a synergistic manner. Both of these aspects in fact help to increase and amplify the process of reducing the local blood pressure, reducing inflammation and pain, and thus accelerating the healing process from accidents.

Advantageously, moreover, the particular morphology of the bands that are interwoven to form a lattice that mimics the morphology of the subcutaneous muscle tissues allows effectively supporting such tissues muscle and at the same time allows for a more effective stimulating action.

In particular, the action of the bands once placed on the skin tissue is to form waves acting on the muscles.

Advantageously, moreover, the use of a silicone-based material to make the bands prevents the risk of problems due to the skin-polymeric material contact. Moreover, such a material contains no drugs or chemical ingredients, is waterproof, can be used for a long period of time and leaves no residues on the skin.

Advantageously, the present invention allows obtaining beneficial effects that tend to be similar to those obtained with neuromuscular taping using adhesive strips, but with the advantage of being easy to wear (even by a non rehabilitation professional) and not to leave residues on the skin.

## Claims

1. Wearable textile article (1) for the assistance of muscle function, comprising:
a tubular element (2) made of a fabric having an inner side (20) suitable for contact with a part of the human or animal body and an outer side (E) opposite the inner side, on said inner side (20) being applied a plurality of bands (200) of polymeric material suitable to be in contact with skin at a contact region of said part of the human or animal body, said bands (200) being arranged along paths (P) suitable to overlap the paths of muscle and/or subcutaneous nerve fibres of said part of the human or animal body when the textile article is worn, so as to create a decompression effect of said body part and a stimulating effect of the muscle and/or nerve fibres in correspondence of the contact region,
**characterized in that** the bands (200) are interconnected so as to form a weave or a lattice
and **in that** the bands (200) comprise a first group of bands (201, 201') comprising a first plurality of bands spaced apart and arranged along first parallel paths (203, 203') and a second group of bands (202, 202') comprising a second plurality of bands spaced apart and arranged along second parallel paths (204, 204'), said second parallel paths (204, 204') being arranged substantially in a specular manner with respect to the first parallel paths with respect to a longitudinal axis of symmetry (Y, Y') parallel to the generatrices of the tubular element.

2. Wearable textile article (1) according to claim 1, wherein the bands (200) run along a curvilinear path having a preferential direction parallel to the direction of the generatrices of the tubular element.

3. Wearable textile article (1) according to claim 1 or 2, wherein the polymeric material of the plurality of bands (200) is a silicone-based material, suitable to ensure the adhesion of the bands to the skin during movement of the body part of the individual.

4. Wearable textile article (1) according to claim 3, wherein the bands (200) extend substantially along the entire height (H) of the tubular element.

5. Wearable textile article (1) according to any of the preceding claims, wherein the bands extend for a given band length (L) from a lower region (22) of the tubular element to an upper region (23) of the tubular element in the direction of the height (H) of the tubular element and for a given transverse width (T) in the direction perpendicular to the length (L), and wherein, in an intermediate region (24) between said lower region (22) and said upper region (23) said bands are tapered towards a median axis (X) incident on the transverse width (T).

6. Wearable textile article (1) according to any one of the preceding claims, wherein the continuity of the bands (200) is interrupted in an interrupted region (400), free from bands (200) and corresponding to the elbow region on the front side when the textile article is worn on an upper limb.

7. Wearable textile article (1) according to claim 6, wherein the interrupted region (400) without bands (200) extends in the direction of the longitudinal axis of symmetry (Y) by a stretch of between 5% and 20% of the total length of the wearable article.

8. Wearable textile article (1) according to any one of claims 1 to 5, wherein the bands (200) are arranged in a region of the tubular element intended to come into contact with a rear region of the leg of an individual, for example the calf.

9. Wearable textile article (1) according to claim 8, wherein the elastic bands (200) are suitable to be arranged in contact only with the rear portion of the leg.

10. Wearable textile article (1) according to any one of the preceding claims, wherein the first (203, 203') and second parallel paths (204, 204') have a curvilinear shape and are intertwined so as to form an overall "X" shape.

11. Wearable textile article (1) according to any one of the preceding claims, wherein the fabric with which the tubular element is made comprises carbon fibres.

12. Wearable textile article (1) according to any one of the preceding claims, wherein the transverse thickness of the bands, measured as the maximum distance between the inner side (20) of the tubular element and the top of each band is comprised between 0.1 mm and 2 mm, preferably 0.4 mm.

13. Wearable textile article (1) according to any one of the claims from 1 to 7 or from 10 to 12, said wearable textile article (1) being a sleeve for an upper limb, such as an elbow compress.

14. Method of manufacture of a wearable textile article according to any of claims 1 to 13, comprising the steps of:
a) providing a tubular element made of fabric;
b) fitting the tubular element on a form where the inner side (20) is exposed towards the outside, said form being suitable to stretch the fabric of the tubular element;
c) printing a desired pattern of bands made of a polymeric material by means of a silk-screen printing technique.

15. Method according to claim 14, wherein step c) comprises:
- positioning a shaped mask according to a negative of the desired pattern for the bands (200);
- spreading the polymeric material in liquid form on the mask, preferably by means of spatula;
- if necessary, removing the mask;
- positioning the wearable article in an oven at a temperature suitable for hardening the polymeric material.

## Patentansprüche

1. Tragbarer Textilartikel (1) zur Unterstützung von Muskelfunktion, umfassend:
ein röhrenförmiges Element (2) aus einem Stoff, der eine Innenseite (20), die zum Kontakt mit einem Teil des menschlichen oder tierischen Körpers geeignet ist, und eine Außenseite (E) gegenüberliegend bzw. entgegengesetzt zu der Innenseite aufweist, wobei auf der Innenseite (20) eine Mehrzahl von Bändern (200) aus Polymermaterial aufgebracht sind, die geeignet sind, mit Haut an einem Kontaktbereich des Teils des menschlichen oder tierischen Körpers in Kontakt zu sein, wobei die Bänder (200) entlang von Bahnen (P) angeordnet sind, die geeignet sind, die Bahnen eines Muskels und/oder von subkutanen Nervenfasern des Teils des menschlichen oder tierischen Körpers zu überlappen, wenn der Textilartikel getragen wird, um einen Dekompressionseffekt des Körperteils und einen stimulierenden Effekt des Muskels und/oder der Nervenfasern in Übereinstimmung mit dem Kontaktbereich zu erzeugen,
**dadurch gekennzeichnet, dass** die Bänder (200) miteinander verbunden sind, um ein Gewebe oder ein Gitter zu bilden,
und dass die Bänder (200) eine erste Gruppe von Bändern (201, 201'), die eine erste Mehrzahl von Bändern umfassen, die voneinander beabstandet sind und entlang erster paralleler Bahnen (203, 203') angeordnet sind, und eine zweite Gruppe von Bänder (202, 202') umfassen, die eine zweite Mehrzahl von Bändern umfassen, die voneinander beabstandet sind und entlang zweiter paralleler Bahnen (204, 204') angeordnet sind, wobei die zweiten parallelen Bahnen (204, 204') im Wesentlichen in spiegelnder Weise in Bezug auf die ersten parallelen Bahnen in Bezug auf eine Längssymmetrieachse (Y, Y') parallel zu den Generatrizen des röhrenförmigen Elements angeordnet sind.

2. Tragbarer Textilartikel (1) nach Anspruch 1, wobei die Bänder (200) entlang einer kurvenförmigen Bahn mit einer Vorzugsrichtung parallel zu der Richtung der Generatrizen des röhrenförmigen Elements verlaufen.

3. Tragbarer Textilartikel (1) nach Anspruch 1 oder 2, wobei das Polymermaterial der Mehrzahl von Bänder (200) ein silikonbasiertes Material ist, das geeignet ist, die Haftung der Bänder an der Haut während der Bewegung des Körperteils des Individuums sicherzustellen.

4. Tragbarer Textilartikel (1) nach Anspruch 3, wobei sich die Bänder (200) im Wesentlichen entlang der gesamten Höhe (H) des röhrenförmigen Elements erstrecken.

5. Tragbarer Textilartikel (1) nach einem der vorhergehenden Ansprüche, wobei sich die Bänder für eine gegebene Bandlänge (L) von einem unteren Bereich (22) des röhrenförmigen Elements zu einem oberen Bereich (23) des röhrenförmigen Elements in der Richtung der Höhe (H) des röhrenförmigen Elements und für eine gegebene Querbreite (T) in der Richtung senkrecht zu der Länge (L) erstrecken, und wobei in einem Zwischenbereich (24) zwischen dem unteren Bereich (22) und dem oberen Bereich (23) die Bänder zu einer Mittelachse (X) verjüngt sind, die auf die Querbreite (T) trifft.

6. Tragbarer Textilartikel (1) nach einem der vorhergehenden Ansprüche, wobei die Kontinuität bzw. Durchgängigkeit der Bänder (200) in einem unterbrochenen Bereich (400) unterbrochen ist, der frei von Bändern (200) ist und dem Ellbogenbereich auf der Vorderseite entspricht, wenn der Textilartikel an einer oberen Extremität getragen wird.

7. Tragbarer Textilartikel (1) nach Anspruch 6, wobei sich der unterbrochene Bereich (400) ohne Bänder (200) in der Richtung der Längssymmetrieachse (Y) um eine Strecke zwischen 5% und 20% der Gesamtlänge des tragbaren Artikels erstreckt.

8. Tragbarer Textilartikel (1) nach einem der Ansprüche 1 bis 5, wobei die Bänder (200) in einem Bereich des röhrenförmigen Elements angeordnet sind, der dazu bestimmt ist, mit einem hinteren Bereich des Beins eines Individuums in Kontakt zu kommen, beispielsweise der Wade.

9. Tragbarer Textilartikel (1) nach Anspruch 8, wobei die elastischen Bänder (200) geeignet sind, nur in Kontakt mit dem hinteren Abschnitt des Beins angeordnet zu sein.

10. Tragbarer Textilartikel (1) nach einem der vorhergehenden Ansprüche, wobei die ersten (203, 203') und die zweiten parallelen Bahnen (204, 204') eine kurvenförmige Form aufweisen und so miteinander verflochten sind, dass sie insgesamt eine "X"-Form bilden.

11. Tragbarer Textilartikel (1) nach einem der vorhergehenden Ansprüche, wobei der Stoff, aus dem das röhrenförmige Element besteht, Kohlenstofffasern umfasst.

12. Tragbarer Textilartikel (1) nach einem der vorhergehenden Ansprüche, wobei die Querdicke der Bänder, gemessen als der maximale Abstand zwischen der Innenseite (20) des röhrenförmigen Elements und der Oberseite jedes Bandes, zwischen 0,1 mm und 2 mm, vorzugsweise 0,4 mm liegt.

13. Tragbarer Textilartikel (1) nach einem der Ansprüche 1 bis 7 oder 10 bis 12, wobei der tragbare Textilartikel (1) ein Ärmel für eine obere Extremität ist, beispielsweise eine Ellbogenkompresse.

14. Verfahren zum Herstellen eines tragbaren Textilartikels nach einem der Ansprüche 1 bis 13, umfassend die Schritte:
a) Bereitstellen eines röhrenförmigen Elements aus Stoff;
b) Anbringen des röhrenförmigen Elements an einer Form, wo die Innenseite (20) zu der Außenseite hin freigelegt ist, wobei die Form geeignet ist, den Stoff des röhrenförmigen Elements zu dehnen;
c) Drucken eines gewünschten Musters von Bändern, die aus einem Polymermaterial bestehen, mittels einer Siebdrucktechnik.

15. Verfahren nach Anspruch 14, wobei Schritt c) umfasst:
- Positionieren einer geformten Maske gemäß einem Negativ des gewünschten Musters für die Bänder (200);
- Verteilen des Polymermaterials in flüssiger Form auf der Maske, vorzugsweise mittels Spatel;
- falls erforderlich, Entfernen der Maske;
- Positionieren des tragbaren Artikels in einem Ofen bei einer Temperatur, die zum Härten des Polymermaterials geeignet ist.

## Revendications

1. Article vestimentaire textile (1) pour l'assistance de la fonction musculaire, comprenant:
un élément tubulaire (2) constitué d'un tissu ayant un côté interne (20) approprié pour le contact avec une partie du corps humain ou animal et un côté externe (E) opposé au côté interne, sur ledit côté interne (20) étant appliquée une pluralité de bandes (200) de matériau polymère approprié pour être en contact avec la peau au niveau d'une région de contact de ladite partie du corps humain ou animal, lesdites bandes (200) étant agencées le long de chemins (P) appropriés pour chevaucher les chemins de fibres musculaires et/ou nerveuses sous-cutanées de ladite partie du corps humain ou animal lorsque l'article textile est porté, de façon à créer un effet de décompression de ladite partie du corps et un effet de stimulation des fibres musculaires et/ou nerveuses en correspondance de la région de contact,
**caractérisé en ce que** les bandes (200) sont interconnectées de façon à former un tissage ou un treillis
et **en ce que** les bandes (200) comprennent un premier groupe de bandes (201, 201') comprenant une première pluralité de bandes espacées et agencées le long de premiers chemins parallèles (203, 203') et un second groupe de bandes (202, 202') comprenant une seconde pluralité de bandes espacées et agencées le long de seconds chemins parallèles (204, 204') , lesdits seconds chemins parallèles (204, 204') étant agencés sensiblement d'une manière spéculaire par rapport aux premiers chemins parallèles par rapport à un axe longitudinal de symétrie (Y, Y') parallèle aux génératrices de l'élément tubulaire.

2. Article vestimentaire textile (1) selon la revendication 1, dans lequel les bandes (200) s'étendent le long d'un chemin curviligne ayant une direction préférentielle parallèle à la direction des génératrices de l'élément tubulaire.

3. Article vestimentaire textile (1) selon la revendication 1 ou 2, dans lequel le matériau polymère de la pluralité de bandes (200) est un matériau à base de silicone, approprié pour assurer l'adhésion des bandes sur la peau pendant un mouvement de la partie du corps de l'individu.

4. Article vestimentaire textile (1) selon la revendication 3, dans lequel les bandes (200) s'étendent sensiblement le long de la totalité de la hauteur (H) de l'élément tubulaire.

5. Article vestimentaire textile (1) selon l'une quelconque des revendications précédentes, dans lequel les bandes s'étendent sur une longueur de bande donnée (L), d'une région inférieure (22) de l'élément tubulaire à une région supérieure (23) de l'élément tubulaire dans la direction de la hauteur (H) de l'élément tubulaire, et sur une largeur transversale donnée (T) dans la direction perpendiculaire à la longueur (L), et dans lequel, dans une région intermédiaire (24) entre ladite région inférieure (22) et ladite région supérieure (23), lesdites bandes se rétrécissent vers un axe médian (X) incident sur la largeur transversale (T).

6. Article vestimentaire textile (1) selon l'une quelconque des revendications précédentes, dans lequel la continuité des bandes (200) est interrompue dans une région interrompue (400), exempte de bandes (200) et correspondant à la région de coude sur le côté avant lorsque l'article textile est porté sur un membre supérieur.

7. Article vestimentaire textile (1) selon la revendication 6, dans lequel la région interrompue (400) sans bandes (200) s'étend dans la direction de l'axe longitudinal de symétrie (Y) sur un étirement d'entre 5 % et 20 % de la longueur totale de l'article vestimentaire.

8. Article vestimentaire textile (1) selon l'une quelconque des revendications 1 à 5, dans lequel les bandes (200) sont agencées dans une région de l'élément tubulaire destinée à venir en contact avec une région arrière de la jambe d'un individu, par exemple le mollet.

9. Article vestimentaire textile (1) selon la revendication 8, dans lequel les bandes élastiques (200) sont appropriées pour être agencées en contact uniquement avec la partie arrière de la jambe.

10. Article vestimentaire textile (1) selon l'une quelconque des revendications précédentes, dans lequel les premier (203, 203') et second chemins parallèles (204, 204') ont une géométrie curviligne et sont entremêlés de façon à former une géométrie globale en « X ».

11. Article vestimentaire textile (1) selon l'une quelconque des revendications précédentes, dans lequel le tissu avec lequel l'élément tubulaire est constitué comprend des fibres de carbone.

12. Article vestimentaire textile (1) selon l'une quelconque des revendications précédentes, dans lequel l'épaisseur transversale des bandes, mesurée comme la distance maximale entre le côté interne (20) de l'élément tubulaire et le dessus de chaque bande, est comprise entre 0,1 mm et 2 mm, de préférence est de 0,4 mm.

13. Article vestimentaire textile (1) selon l'une quelconque des revendications 1 à 7 ou 10 à 12, ledit article vestimentaire textile (1) étant un manchon pour un membre supérieur, tel qu'une compresse de coude.

14. Procédé de fabrication d'un article vestimentaire textile selon l'une quelconque des revendications 1 à 13, comprenant les étapes de :
a) la fourniture d'un élément tubulaire constitué de tissu ;
b) l'ajustement de l'élément tubulaire sur une forme où le côté interne (20) est exposé vers l'extérieur, ladite forme étant appropriée pour étirer le tissu de l'élément tubulaire ;
c) l'impression d'un motif souhaité de bandes constituées d'un matériau polymère au moyen d'une technique de sérigraphie.

15. Procédé selon la revendication 14, dans lequel l'étape c) comprend :
- le positionnement d'un masque façonné selon un négatif du motif souhaité pour les bandes (200) ;
- l'étalement du matériau polymère sous forme liquide sur le masque, de préférence au moyen d'une spatule ;
- si nécessaire, le retrait du masque ;
- le positionnement de l'article vestimentaire dans un four à une température appropriée pour durcir le matériau polymère.
